# EUROPÄISCHE PATENTSCHRIFT

(11) **EP 2 186 498 B1**
(45) Veröffentlichungstag und Bekanntmachung des Hinweises auf die Patenterteilung: **27.04.2011**
(21) Anmeldenummer: 09168488.6
(22) Anmeldetag: 24.08.2009
(51) Int. Cl.: A61G 7/018, A61N 5/10, A61B 6/04, A61G 7/002

(54) **Patiententransporteinheit und Verfahren zum Transport eines Patienten**
Patient transport unit and method for transporting a patient
Unité de transport de patients et procédé de transport d'un patient

(30) Priorität: 13.11.2008 DE 102008057145
(43) Veröffentlichungstag der Anmeldung: 19.05.2010
(73) Patentinhaber: SIEMENS AKTIENGESELLSCHAFT, 80333 München (DE)
(72) Erfinder: Use, Tim, 90469, Nürnberg (DE); Höppner, Claus-Peter, 91301, Forchheim (DE)

(56) Entgegenhaltungen:
- GB-A- 2 278 264
- US-A- 3 814 414
- US-A- 5 595 192
- US-A1- 2008 098 525

## Beschreibung

Die Erfindung betrifft ein im Patentanspruch 1 angegebenes Verfahren zur Beförderung eines Patienten.

Als Strahlentherapie wird im Allgemeinen die Anwendung ionisierender Strahlung in der Heilkunde bezeichnet. Dabei wird hochenergetische Strahlung (Röntgenstrahlung, Gammastrahlung) oder Partikelstrahlung (Elektronen, Protonen, Kohlenstoffionen, etc.) auf einen zu behandelnden Körperbereich eines Patienten gelenkt. Eine Strahlenapplizierung kann aber auch in nicht-therapeutischen Bereichen eingesetzt werden, wie beispielsweise bei der Bestrahlung von Phantomen oder nichtlebenden Körpern im Rahmen von Forschungsarbeiten, oder bei der Bestrahlung von Materialen.

Für die Partikeltherapie wird hochenergetische Partikelstrahlung mit einer Beschleunigungsanlage erzeugt. Die auf hohe Energien beschleunigten Partikel werden zu einem Partikelstrahl geformt und anschließend auf das zu bestrahlende Gewebe gerichtet. Die Partikel dringen in das zu bestrahlende Gewebe ein und geben dort in einem umschriebenen Bereich ihre Energie ab. Die Eindringtiefe des Partikelstrahls in das zu bestrahlende Gewebe hängt vornehmlich von der Energie des Partikelstrahls ab. Je höher die Energie des Partikelstrahls, desto tiefer dringen die Partikel in das zu bestrahlende Gewebe ein. Im Vergleich zu herkömmlichen Bestrahlungsverfahren, die mit Röntgenstrahlen arbeiten, zeichnet sich die Partikeltherapie dadurch aus, dass die Energie der Partikel in einem umschriebenen und abgrenzbaren Bereich abgegeben wird. Hierdurch kann eine Bestrahlung z.B. eines Tumors genauer erfolgen und umliegendes Gewebe kann besser geschont werden. Die Partikeltherapie wird üblicherweise in einer speziellen Partikeltherapieanlage durchgeführt, in der einerseits in einem Bereich der Partikelstrahl erzeugt und in der Regel zu mehreren Bestrahlungsräumen geführt wird, und in der andererseits in einem anderen Bereich verschiedene Räume vorhanden sind, in denen Patienten für eine bevorstehende Bestrahlungssitzung vorbereitet oder während einer Bestrahlungssitzung bestrahlt werden.

Dokument GB2278264 offenbart eine Transporteinheit mit einer Lagerungsvorrichtung zur Beförderung eines Patienten.

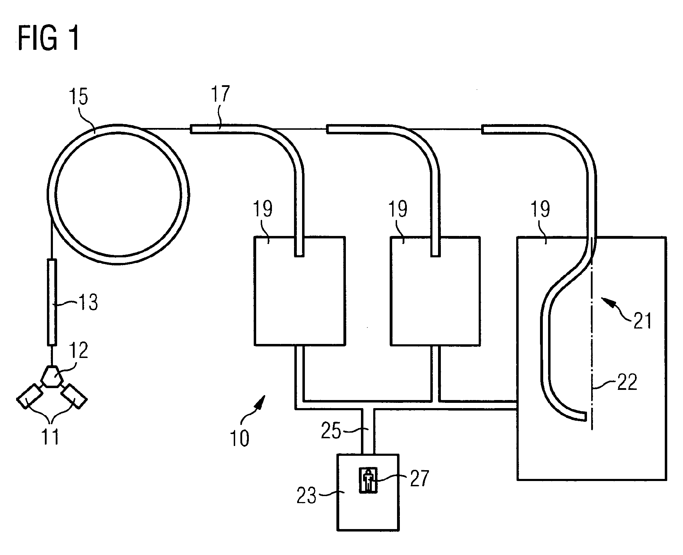

Figur 1 zeigt eine schematischen Übersicht eines Aufbaus einer Partikeltherapieanlage 10 gemäß der nachveröffentlichten DE 10 2008 005 068 A1. Als Partikel werden vornehmlich Ionen wie beispielsweise Protonen, Heliumionen oder Kohlenstoffionen eingesetzt. Die Partikel werden in einer Partikelquelle 11 erzeugt. Wenn, wie in Figur 1 dargestellt, zwei Partikelquellen 11 vorhanden sind, die zwei verschiedene Ionenarten erzeugen, kann zwischen diesen beiden Ionenarten innerhalb kürzester Zeit umgeschaltet werden. Dazu wird beispielsweise ein Schaltmagnet 12 verwendet, der zwischen den Ionenquellen 11 einerseits und einem Vorbeschleuniger 13 andererseits angeordnet ist. Dadurch kann die Partikeltherapieanlage 10 mit Protonen und mit Kohlenstoffionen gleichzeitig betrieben werden. Dukument G82278264 offenbard eine Transportteinleit mid einer Lagerungsvomichtung zurBeförderung einses Parienton.

Die von der oder einer der Ionenquellen 11 erzeugten und gegebenenfalls mit dem Schaltmagneten 12 ausgewählten Ionen werden in dem Vorbeschleuniger 13 auf ein erstes Energieniveau beschleunigt. Der Vorbeschleuniger 13 ist beispielsweise ein Linearbeschleuniger. Anschließend werden die Partikel in einen Beschleuniger 15, beispielsweise ein Synchrotron, eingespeist. In dem Beschleuniger 15 werden sie auf hohe Energien, wie sie zur Bestrahlung nötig sind, beschleunigt. Nachdem die Partikel den Beschleuniger 15 verlassen haben, führt ein Hochenergiestrahl-Transportsystem 17 den Partikelstrahl zu einem oder mehreren Bestrahlungsräumen 19. In einem Bestrahlungsraum 19 werden die beschleunigten Partikel auf einen zu bestrahlenden Körperteil gerichtet. Je nach Ausgestaltung erfolgt dies von einer festen Richtung aus oder aber über eine um eine Achse 22 bewegliche rotierbare Gantry 21 von verschiedenen Richtungen aus.

Weiterhin weist die Partikeltherapieeinrichtung 10 verschiedene weitere Räume 23 auf, in denen beispielsweise Patienten auf eine bevorstehende Bestrahlungssitzung bzw. für eine bevorstehende Untersuchung vorbereitet werden. Diese weiteren Räume 23 sowie die Bestrahlungsräumen 19 stehen untereinander über Korridore 25 in Verbindung. Dabei tritt oft der Fall ein, dass ein Patient in einem Raum vorbereitet wird und anschließend in einen anderen Raum gebracht werden muss. Die Vorbereitung schließt in der Regel eine Positionierung eines Patienten auf einer Transporteinheit 27 ein, so dass der auf der Transporteinheit 27 positionierte Patient anschließend in einem anderen Raum gefahren werden kann. Die Transporteinheit 27 ist dabei sowohl eine Patientenlagerungsvorrichtung als auch eine Patiententransportvorrichtung, da ein Patient auf der Transporteinheit sowohl gelagert als auch mit der Transporteinheit von Raum zu Raum transportiert wird.

Werden Patienten auf eine Bestrahlungssitzung vorbereitet, werden sie üblicherweise auf eine Patientenhalterungsvorrichtung positioniert und teilweise auch fixiert, damit später in einem Bestrahlungsraum eine genaue Ausrichtung des Patienten in Bezug auf den Partikelstrahl erfolgen kann. Um die Behandlung der Patienten möglichst effektiv zu gestalten, werden hierfür Patiententransporteinheiten eingesetzt. Derartige Transporteinheiten sind Patientenlagerungs- und Patiententransportvorrichtungen, auf denen ein Patient einerseits gelagert und gegebenenfalls fixiert werden kann, und mit denen andererseits ein Patient anschließend von einem Raum in einen anderen Raum - beispielsweise von einem Vorbereitungsraum in einen Bestrahlungsraum - gefahren werden kann.

Im Vorbereitungsraum kann gegebenenfalls die Patientenposition und insbesondere die Position des zu bestrahlenden Körperbereichs des Patienten mittels bildgebender medizinischer Methoden verifiziert werden. Hierzu werden beispielsweise röntgentomografische Aufnahmemethoden, wie die Computertomografie, eingesetzt. In der Regel wird mit einem Datensatz aus der Computertomografie geplant. Dabei ist der Patient auf einem Tisch positioniert, dessen Tischplatte möglichst horizontal ausgerichtet ist.

Bei der Planung kann sich aber ergeben, dass eine Behandlung in einer gekanteten und/oder gekippten Lage günstig wäre. Dabei stellt sich folgendes Problem: durch die Änderung der Körperlage ändern sich auch die Lagen der inneren Organe und somit ergibt sich eine Diskrepanz zwischen der ursprünglichen Planung und der aktuellen Lage des Patienten. Dieses Problem kann durch die Verwendung eines Roboters bei der Planungscomputertomografie und mit einem Roboter in der Behandlung gelöst werden, wobei die Roboter die Liegflächen entsprechend drehen.

Vor einer Strahlenbehandlung wird der Patient in einem Vorbereitungsraum für die Behandlung vorbereitet. Dazu wird er auf einen horizontalen Tisch gelegt. Zur Erhöhung der Behandlungsgenauigkeit kann eine Röntgenaufnahme erfolgen. Dies wird je nach Ausstattung der Anlage in den Behandlungsräumen selbst oder außerhalb in einem separaten CT-Raum durchgeführt. Im zweiten Fall muss der Patient nach der Positionsverifikation zunächst in den Behandlungsraum befördert werden, bevor die Behandlung erfolgen kann.

Nach einer Positionsverifikation außerhalb des Behandlungsraumes in einer aus der Planung ermittelten Kipp-/Kantposition ergeben sich durch den Transport des Patienten in Horizontalposition aus dem Bildgebungs-/Vorbereitungsraum in den Behandlungsraum Verschiebungen der Organe und des Tumors, und somit Fehler in der Bestrahlung.

Es ist daher die Aufgabe der Erfindung eine Transporteinheit und ein Verfahren zum Transport von Patienten anzugeben, bei denen es zu keiner Verschiebung der Organe durch einen Patiententransport kommt.

Gemäß der Erfindung wird die gestellte Aufgabe mit dem Transportverfahren des unabhängigen Patentanspruchs 1 gelöst.

Die Erfindung beansprucht eine Transporteinheit mit einer Lagerungsvorrichtung zur Beförderung eines Patienten von einem ersten Raum in mindestens einen zweiten Raum. Sie umfasst mindestens ein erstes Mittel, das mit der Lagerungsvorrichtung zusammenwirkt, um die Beförderung des Patienten vom ersten in den zweiten Raum in einer gekippten und/oder gekanteten Position durchzuführen.

Die Erfindung bietet eine Vielzahl von Vorteilen. Die Positionsverifikation kann außerhalb eines Behandlungsraumes erfolgen und ermöglicht:
a) die Verwendung eines Computertomografiegeräts mit einer verbesserten Auflösung gegenüber einem Standard Robotimager System, wie es gegenwärtig in den Behandlungsräumen geplant ist,
b) kleinere Behandlungsräume gegenüber Planungen, in denen ein Computertomograph im Behandlungsraum untergebracht werden soll und
c) eine bessere zeitliche Nutzung von Behandlungsräumen, da der Vorgang der Positionsverifikation in einem anderen Raum stattfindet und der Behandlungsraum zwischenzeitlich für eine andere Behandlung genutzt werden kann.

Durch die frühzeitige Positionierung des Patienten in seiner Planungs- und Behandlungsposition können die Planung und vor allem aber auch die Behandlung mit einer höheren Präzision erfolgen.

In einer Weiterbildung kann die Transporteinheit eine Beförderungsvorrichtung zur Aufnahme und Beförderung der Lagerungsvorrichtung umfassen. Dadurch ist die Lagerungsvorrichtung von der Beförderungsvorrichtung trennbar.

In einer weiteren Ausführungsform kann das erste Mittel ein Teil der Beförderungsvorrichtung sein. Dies bietet den Vorteil, dass Höhenverstellungen auch ohne Lagerungsvorrichtung vorgenommen werden können.

Des Weiteren kann die Transporteinheit mindestens ein zweites Mittel umfassen, das mit der Lagerungsvorrichtung derart zusammenwirkt, dass der Patient auf der Lagerungsvorrichtung immobilisiert werden kann. Dadurch behalten der Patient und seine Organe die eingenommene und eingestellte Lage auch bei einer Beförderung bei.

In bevorzugter Weise kann das erste Mittel zwei vertikal verfahrbare erste Träger und zwei zweite Träger umfassen, wobei die zweiten Träger jeweils auf einem ersten Träger drehbar gelagert sind, die Lagerungsvorrichtung auf den zweiten Trägern lösbar angeordnet ist und durch eine unterschiedliche Höhenverstellung der ersten Träger das Kippen und durch Drehen der zweiten Träger das Kanten erfolgt. Vorteilhaft daran ist ein robuster und einfacher Einstellprozess.

In einer bevorzugten Ausführungsform kann die Lagerungsvorrichtung an ihrer Unterseite in mindestens drei Ecken Halbschalenelemente umfassen, in die korrespondierende Kugelköpfe höhenverstellbarer dritter Träger derart greifen, dass infolge unterschiedlicher Höhen der Kugelköpfe die Lagerungsvorrichtung gekippt und/oder gekantet werden kann. Dadurch kann die Lagerungsvorrichtung einfach und sicher verstellt werden.

In einer Weiterbildung kann die Transporteinheit ein erstes Haltemodul umfassen, mit dessen Hilfe ein Roboterarm die Lagerungsvorrichtung aufnehmen kann. Dies bietet den Vorteil, dass die Lagerungsvorrichtung sicher und präzise von einer Roboteranlage übernommen werden kann.

Des Weiteren kann die Transporteinheit ein zweites Haltemodul umfassen, mit dessen Hilfe die Lagerungsvorrichtung lösbar fest an der Beförderungsvorrichtung fixiert werden kann, wobei die Fixierung bei Übernahme der Lagerungsvorrichtung durch einen Roboterarm automatisch gelöst wird. Die Lagerungsvorrichtung ist dadurch während einer Bewegung sicher mit der Beförderungsvorrichtung verbunden und kann trotzdem automatisch von einer Roboteranlage übernommen werden.

Außerdem kann die Transporteinheit eine Anzeigeeinheit umfassen, die aktuelle Werte der Kantung und Kippung, Sollwerte der Kantung und Kippung und/oder Abweichungen der aktuellen Werte von den Sollwerten ausgibt. Dadurch kann ein Bedienpersonal jederzeit den Status der Orientierung der Lagerungsvorrichtung erkennen.

In einer weiteren Ausführungsform kann die Tranporteinheit einen elektromechanischen Antrieb und dazugehörige Bedienelemente umfassen, die das Kippen und/oder Kanten bewirken. Dies bietet eine sichere, für das Bedienpersonal kräfteschonende Bedienung.

In einer Weiterbildung kann die Transporteinheit einen in der Beförderungseinheit angeordneten Akkumulator zur Energieversorgung des elektromechanischen Antriebs umfassen. Vorteilhaft daran ist die Unabhängigkeit von einer stationären Stromversorgung.

Die Erfindung beansprucht auch die Verwendung einer erfindungsgemäßen Transporteinheit zur Beförderung von immobilisierten Patienten zwischen Räumen einer Strahlentherapieanlage. Dies bietet den Vorteil einer exakten Strahlenbehandlung in gekippter und gekanteter Patientenlage.

Die Erfindung beansprucht des Weiteren auch ein Verfahren zur Beförderung eines Patienten von einem ersten Raum in mindestens einen zweiten Raum mit folgenden Schritten:
a) Immobilisierung des Patienten auf einer Lagerungsvorrichtung,
b) Kippen und Kanten der Lagerungsvorrichtung in eine vorgebbare Position und
c) Beförderung der Lagerungsvorrichtung in der vorgebbaren Position vom ersten Raum in den zweiten Raum.

In einer Weiterbildung kann der erste Raum ein Vorbereitungsraum und der zweite Raum ein Bestrahlungsraum einer Strahlentherapieanlage sein. Dadurch bleiben nach einer Vorbereitung eines Patienten seine Organe in unveränderter Position.

In einer bevorzugten Ausführungsform kann die Beförderung der Lagerungsvorrichtung auf einer verfahrbaren Beförderungsvorrichtung ausgeführt werden, wobei das Kippen und Kanten der Lagerungsvorrichtung durch die Beförderungsvorrichtung erfolgt.

Des Weiteren kann das Verfahren folgenden zusätzlichen Schritt umfassen:
d) Übernahme der Lagerungsvorrichtung von der Beförderungsvorrichtung durch ein Roboterpositionierungssystem.

Weitere Besonderheiten und Vorteile der Erfindung werden aus den nachfolgenden Erläuterungen mehrerer Ausführungsbeispiele anhand von schematischen Zeichnungen ersichtlich.

Es zeigen:
- Figur 1:: eine Übersicht einer Partikeltherapieanlage,
- Figur 2:: eine perspektivische Ansicht einer Transportein- heit mit verfahrbaren Trägern,
- Figur 3:: eine perspektivische Ansicht einer Transportein- heit mit Kugelköpfen und
- Figur 4:: ein Ablaufdiagramm eines Beförderungsverfahrens.

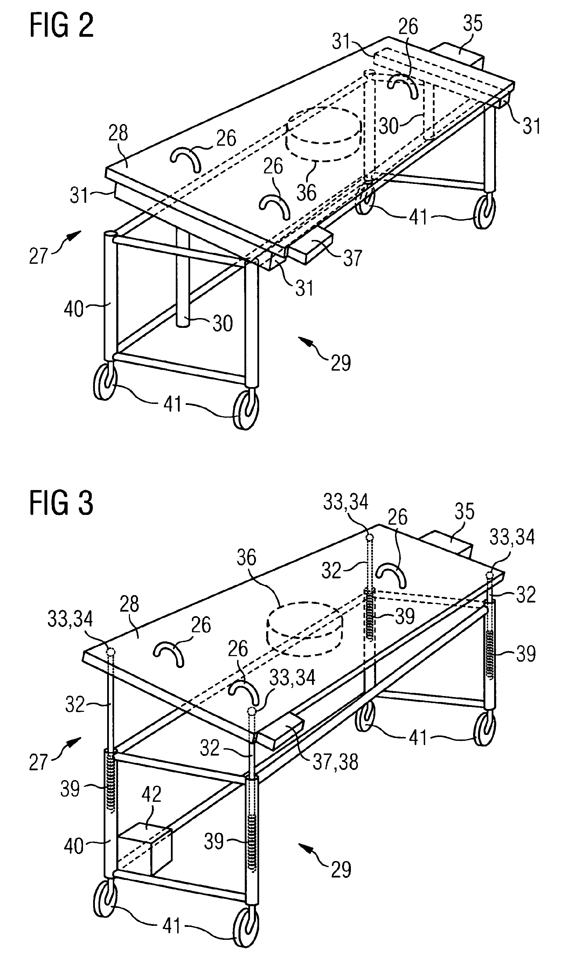

Figur 2 zeigt eine perspektivische Ansicht einer Transporteinheit 27 zur Beförderung von Patienten von einem Raum einer Strahlenbehandlungsanlage in einen weiteren Raum der Strahlenbehandlungsanlage. Bei der Strahlenbehandlungsanlage handelt es sich bevorzugt um eine Partikeltherapieanlage. Die Transporteinheit 27 wird auch als Patientenshuttle bezeichnet.

Die Transporteinheit 27 umfasst eine Beförderungsvorrichtung 29 auf der eine Lagerungsvorrichtung 28 angeordnet ist. Beide Vorrichtungen sind mit Hilfe eines zweiten Haltemoduls 36 zueinander beweglich miteinander lösbar verbunden. Die Beförderungsvorrichtung 29 umfasst eine Gestelleinheit 40 und vier unterhalb der Gestelleinheit angeordnete Räder 41 zum Verfahren der Transporteinheit 27. In einer speziellen Ausführungsform können die Räder 41 als Schienenräder zum Rollen auf einem Schienensystem ausgebildet sein.

Um die räumliche Orientierung der Lagerungsvorrichtung 28 parallel zu einer Bodenoberfläche zu verändern, sind zwei erste Träger 30 jeweils an einem Ende der Gestelleinheit 40 höhenverstellbar angebracht. Auf den beiden Trägern 30 sind drehbar gelagerte zweite Träger 31 in einer Ausgangsposition horizontal zur Bodenoberfläche angeordnet. Auf den beiden zweiten Trägern 31 liegt die Lagerungsvorrichtung 28. Durch eine ungleiche Höhenverstellung der beiden ersten Träger 30 wird die Lagerungsvorrichtung 28 gekippt, das heißt um ihre Querachse gedreht, und durch eine Drehbewegung der zweiten Träger 31 wird die Lagerungsvorrichtung 28 gekantet, das heißt um ihre Längsachse gedreht. Das Kippen wird auch als "Roll", das Kanten auch als "Tilt" oder "Pitch" bezeichnet.

Mit Hilfe von zweiten Mitteln 26 kann ein Patient auf der Lagerungsvorrichtung 28 immobilisiert werden, das heißt er ist in einer für eine Behandlung bevorzugte Lage fixiert. Somit kann der Patient in gekippter und gekanteter Lage von beispielweise einem Vorbereitungsraum der Partikeltherapieanlage in einen Behandlungsraum bzw. Bestrahlungsraum der Partikeltherapieanlage transportiert werden. Auf einer an der Beförderungsvorrichtung 29 montierten Anzeigeeinheit 37 können die unterschiedlichen Neigungswinkel der Kippung und Kantung ausgegeben werden. Die Höhenverstellung der ersten Träger 30 und die Neigung der zweiten Träger 31 können mittels nicht dargestellter mechanischer Kurbeln von Hand aus erfolgen. Ein mit der Lagerungsvorrichtung 28 verbundenes erstes Haltemodul 35 dient als Angriffspunkt eines Roboterarms, um die Lagerungsvorrichtung 28 von der Beförderungsvorrichtung 29 abzuheben Figur 3 zeigt eine perspektivische Ansicht einer weiteren Transporteinheit 27 zur Beförderung von Patienten von einem Raum einer Strahlenbehandlungsanlage in einen weiteren Raum der Strahlenbehandlungsanlage. Die Transporteinheit 27 umfasst eine Beförderungsvorrichtung 29 auf der eine Lagerungsvorrichtung 28 angeordnet ist. Beide Vorrichtungen sind mit Hilfe eines zweiten Haltemoduls 36 zueinander beweglich miteinander lösbar verbunden. Die Beförderungsvorrichtung 29 umfasst eine Gestelleinheit 40 und vier unterhalb der Gestelleinheit 40 angeordnete Räder 41 zum Verfahren der Transporteinheit 27.

Um die räumliche Orientierung der Lagerungsvorrichtung 28 parallel zu einer Bodenoberfläche zu verändern, sind in den vier Ecken der Unterseite der Lagerungsvorrichtung 28 halbschalenförmige Öffnungen 33 angebracht, in die korrespondierende Kugelköpfe 34 von höhenverstellbaren dritten Trägern 32 passgenau greifen. Die vier dritten Träger 32 sind über einen elektromechanischen Antrieb 39 mit der Gestelleinheit 40 verbunden. Der elektromechanische Antrieb wird von einem Akkumulator 42 der Beförderungsvorrichtung 29 mit elektrischer Energie versorgt. Mittels eines Bedienelements können die Höhen der Kugelköpfe 34 gesteuert werden. Durch unterschiedliche Höhen der Kugelköpfe 34 lässt sich die Lagerungsvorrichtung 28 gegenüber einer Bodenoberfläche kippen und /oder kanten. Mit Hilfe der zweiten Mittel 26 kann ein Patient auf der Lagerungsvorrichtung 28 immobilisiert werden. Ein erstes Haltemodul 35 dient als Angriffspunkt eines Roboterarms, um die Lagerungsvorrichtung 28 von der Beförderungsvorrichtung 29 abzuheben.

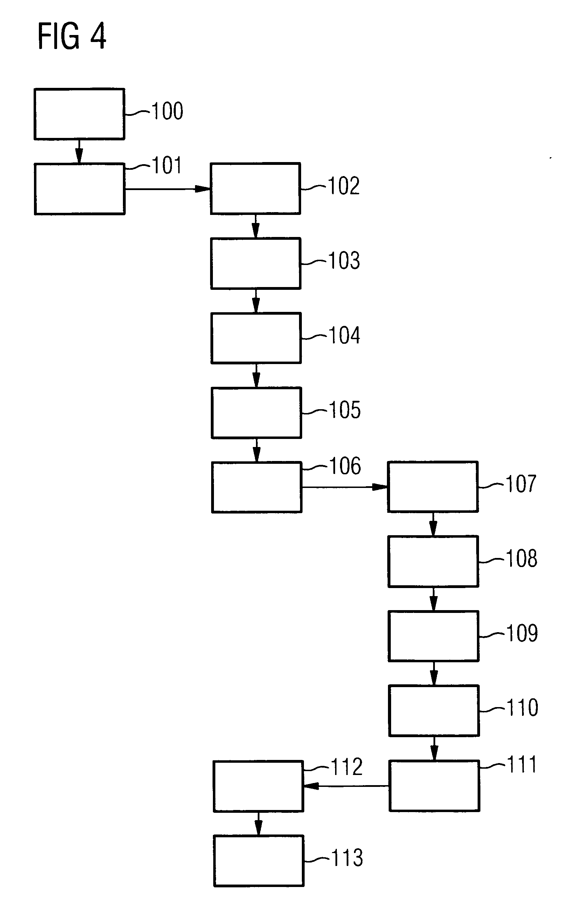

Figur 4 zeigt ein Ablaufdiagramm eines erfindungsgemäßen Verfahrens zur Beförderung eines Patienten von einem Vorbereitungsraum in einen Behandlungsraum. Das Verfahren umfasst folgende Schritte 100 bis 113:
- 100: Planung einer Behandlungsregion, wobei eine Patientenla- gerungsvorrichtung auch Kant- und/oder Kipp-Positionen einnehmen kann,
- 101: Immobilisierung des Patienten auf einer Transporteinheit
- 102: Beförderung des Patienten auf der Transporteinheit in den Vorbereitungsraum zur Positionsverifikation, vorzugsweise mit einer Computertomografieanlage mit Robotersystem zur Positionierung der Patientenlagerungsvorrichtung,
- 103: Einstellen der Patientenposition mit dem Roboter auch in Kant- und/oder Kipp-Winkeln,
- 104: Positionsverifikation in der Behandlungsposition,
- 105: Einstellen der Kant- und/oder Kipp-Winkel an einer Beför- derungsvorrichtung der Transporteinheit,
- 106: Übergabe des Patientenlagerungsvorrichtung vom Roboter an die Beförderungseinheit, wobei die Kant- und/oder Kipp- Position der Lagerungsvorrichtung beibehalten wird,
- 107: Beförderung des Patienten mit der Transporteinheit in den Behandlungsraum,
- 108: Übernahme des Patienten auf der Lagerungsvorrichtung von einem Roboterpositioniersystem im Behandlungsraum,
- 109: Positionierung des Patienten in der Behandlungsposition vor einem Partikelstrahlausgang,
- 110: Behandlung des Patienten,
- 111: Ablegen des Patienten mit der Lagerungsvorrichtung auf die Beförderungseinheit in horizontaler Position oder Ab- legen des Patienten in der Kipp- und Kantposition und Verfahren der Lagerungsvorrichtung in eine horizontale Position,
- 112: Beförderung des Patienten aus dem Behandlungsraum und
- 113: Mobilisierung des Patienten.

Zwischen Schritt 100 und 101 können auch mehrere Tage liegen. Die Patientenpositionierung und Bildgebung können auch im selben Raum erfolgen.

Die oben beschriebene Vorrichtung und das Verfahren können entsprechend auch in einer Bestrahlungseinrichtung mit ionisierender Strahlung verwendet werden.

### Bezugszeichenliste

- 10: Partikeltherapieanlage
- 11: Partikelquelle
- 12: Schaltmagnet
- 13: Vorbeschleuniger
- 15: Beschleuniger
- 17: Hochenergiestrahl-Transportsystem
- 19: Bestrahlungsraum / Behandlungsraum
- 21: Gantry
- 22: Achse
- 23: Vorbereitungsraum
- 25: Korridor
- 26: zweites Mittel
- 27: Transporteinheit / Patientenshuttle
- 28: Lagerungsvorrichtung
- 29: Beförderungsvorrichtung
- 30: erster Träger
- 31: zweiter Träger
- 32: dritter Träger
- 33: Halbschalenelement
- 34: Kugelkopf
- 35: erstes Haltemodul
- 36: zweites Haltemodul
- 37: Anzeigeeinheit
- 38: Bedienelement
- 39: Elektromechanischer Antrieb
- 40: Gestelleinheit
- 41: Rad
- 42: Akkumulator

## Patentansprüche

1. Verfahren zur Beförderung eines Patienten von einem Vorbereitungsraum (23) in einen Bestrahlungsraum (19) einer Strahlentherapieeinrichtung,
**gekennzeichnet durch**:
a) Immobilisierung (101) des Patienten auf einer Lagerungsvorrichtung (28),
b) Beförderung (102) des Patienten auf der Lagerungsvorrichtung (28) in den Vorbereitungsraum,
c) Kippen und/oder Kanten (103) der Lagerungsvorrichtung (28) in eine Behandlungsposition,
d) Positionsverifikation (104) eines zu bestrahlenden Körperbereichs in der Behandlungsposition,
e) Einstellen der Kippung und Kantung (105) an einer Beförderungsvorrichtung (29),
g) Übergabe (106) der Lagerungsvorrichtung (28) an die Beförderungsvorrichtung (29), wobei die Kantung und Kippung der Behandlungsposition beibehalten wird und
f) Beförderung (107) der Beförderungsvorrichtung zusammen mit der Lagerungsvorrichtung (28) in der Behandlungsposition vom Vorbereitungsraum (23) in den Bestrahlungsraum (19).

2. Verfahren nach Anspruch 1,
**dadurch gekennzeichnet,**
**dass** die Positionsverifikation (104) mittels Computertomografie durchgeführt wird.

3. Verfahren nach Anspruch 1 oder 2,
**gekennzeichnet durch**:
g) Übernahme (108) der Lagerungsvorrichtung (28) von der Beförderungsvorrichtung (29) **durch** ein Roboterpositionierungssystem.

4. Verfahren nach Anspruch 3,
**gekennzeichnet durch**:
h) Positionierung (109) des Patienten in der Behandlungsposition vor einem Partikelstrahlenausgang.

## Claims

1. Method for conveying a patient from a preparation room (23) into an irradiation room (19) of a radiotherapy device. **characterised by**:
a) Immobilisation (101) of the patient on a support facility (28),
b) Conveying (102) the patient on the support facility (28) into the preparation room,
c) Tilting and/or canting (103) of the support facility (28) into a treatment position
d) Position verification (104) of an area of the body to be irradiated in the treatment position,
e) Adjusting the tilting and canting (105) on a conveyance facility (29)
g) Transfer (106) of the support facility (28) to the conveyance facility (29), with the tilting and canting of the treatment position being retained and
f) Conveying (107) the conveyance facility together with the support facility (28) in the treatment position from the preparation room (23) into an irradiation room (19).

2. Method according to claim 1,
**characterised in that**
the position verification (104) is undertaken by computer tomography.

3. Method according to claim 1 or 2,
**characterised by**:
g) Acceptance (108) of the support facility (28) from the conveyance facility (29) by a robot positioning system.

4. Method according to claim 3,
**characterised by**:
h) Positioning (109) of the patient in the treatment position before a particle irradiation.

## Revendications

1. Procédé de transport d'un patient d'une salle de préparation (23) dans une salle d'irradiation (19) d'une installation de radiothérapie,
**caractérisé par** :
a) immobilisation (101) du patient sur un dispositif d'installation (28),
b) transport (102) du patient sur le dispositif d'installation (28) dans la salle de préparation,
c) basculement et/ou pliage (103) du dispositif d'installation (28) dans une position de traitement,
d) vérification de position (104) d'une région du corps à irradier dans la position de traitement,
e) réglage du basculement et pliage (105) sur un dispositif de transport (29),
g) transfert (106) du dispositif d'installation (28) au dispositif de transport (29), le pliage et basculement de la position de traitement étant conservés, et
f) transport (107) du dispositif de transport ensemble avec le dispositif d'installation (28) dans la position de traitement de la salle de préparation (23) dans la salle d'irradiation (19).

2. Procédé selon la revendication 1,
**caractérisé en ce que**
la vérification de position (104) est effectuée par tomographie par ordinateur.

3. Procédé selon la revendication 1 ou 2,
**caractérisé par** :
g) reprise (108) du dispositif d'installation (28) du dispositif de transport (29) par un système de positionnement robotisé.

4. Procédé selon la revendication 3,
**caractérisé par** :
h) positionnement (109) du patient dans la position de traitement devant une sortie de faisceaux de particules.
